# EUROPEAN PATENT SPECIFICATION

(11) **EP 1 164 928 B1**
(45) Date of publication and mention of the grant of the patent: **01.06.2005**
(21) Application number: 01900586.7
(22) Date of filing: 20.01.2001
(51) Int. Cl.: A61B 5/04, A61M 5/32

(54) **METHOD OF FABRICATING A MEDICAL ELECTRODE**
HERSTELLUNGSVERFAHREN FÜR EINE MEDIZINISCHE ELEKTRODE
METHODE DE FABRICATION D'UNE ELECTRODE MEDICALE

(30) Priority: 21.01.2000 US 177423 P; 21.01.2000 US 489556
(43) Date of publication of application: 02.01.2002
(73) Proprietor: INSTRUMENTARIUM CORPORATION, 00510 Helsinki (FI)
(72) Inventor: Stemme, Goran, 114 21 Stockholm (SE); Enoksson, Jan Peter, 112 26 Stockholm (SE); Griss, Patrick, 6014 Littau (CH); Merilainen, Pekka, 00660 Helsinki (FI)
(74) Representative: Charlton, Peter John
(86) International application number: PCT/IB2001/000059
(87) International publication number: WO 2001/052731

(56) References cited:
- EP-A- 0 571 120
- WO-A-97/03718
- US-A- 4 004 578
- US-A- 5 305 746
- HENRY S ET AL: "MICROMACHINED NEEDLES FOR THE TRANSDERMAL DELIVERY OF DRUGS" IEEE WORKSHOP ON MICRO ELECTRO MECHANICAL SYSTEMS,US,NEW YORK, NY: IEEE, 25 January 1998 (1998-01-25), pages 494-498, XP000829211 ISBN: 0-7803-4413-8
- NORMANN ET. AL: "Micromachined, Silicon based electrode arrays for electrical stimulation of or recording from cerebral cortex" PROC. MICRO ELECTRO MECHANICAL SYTEMS WORKSHOP, 1991, pages 247-252, XP002166861 Nara, Japan
- GRISS ET. AL.: "Spiked biopotential electrodes" PRPCEEDINGS IEEE THIRTEENTH ANNUAL INTERNATIONAL CONFERENCE ON MICRO ELECTRO MECHANICAL SYSTEMS , 23 - 27 January 2000, pages 323-328, XP002166862 Miyazaki, Japan

## Description

### BACKGROUND OF THE INVENTION

The present invention relates to an electrode suitable for application to the skin of a living subject to obtain biopotentials and/or provide electrostimulation.

Skin applied electrodes measuring biopotentials (i.e. biopotential electrodes) are extensively used in modem clinical and biomedical applications (e.g. electrocardiography, electromyography, electroencephalography (EEG), peripheral nerve compound action potentials, and evoked potentials). The characteristics of the electrodes are very important since measurement electronics equipment coupled to the electrodes is likely to display artifacts or misleading data if an inappropriate electrode is used.

Important drawbacks of electrodes currently in use relate to skin preparation and the use of electrolytic gel (i.e. wet use). Long application times, long stabilization times required for the diffusion of the electrolytic gel into the skin, long cleansing times to remove the gel after use, low comfort, and large electrode size complicate the use of standard electrodes. Research into eliminating or reducing the inconveniences of standard electrodes has lead to several new approaches to measuring biopotentials including Nasicon ceramic electrodes, on chip amplified dry electrodes, and the electrode sold under the trademark Zipprep. See *Non-Polarisable Dry Electrode Based on NASICON Ceramic*, Ch. Gondran et al., Medical & Biological Engineering & Computing, May 1995; *An Active, Microfabricated, Scalp Electrode-Array for EEG Recording,* Babak Alizadeh-Taheri, Rosemary L. Smith, and Robert T. Knight, Sensors and Actuators A54, Elsevier Science S.A., 1996; and *Low-cost Active Electrode Improves the Resolution in Biopotential Recordings*, Alexander C. Metting VanRijn, Anthony P. Kuiper, Taco E. Dankers and Cees A. Grimpergen, 18th Int. Conf. of the IEEE Engineering in Medicine and Biology Society, Amsterdam, 1996.

Biopotentials result from the electrochemical activity of a certain class of cells, known as excitable or active cells, which are part of nervous, muscular or glandular tissue. Within a human body, active cells are surrounded by body fluids having a high Cl⁻ concentration. An active cell acts as a constant current source when stimulated and creates an ionic current within the body fluid. This current induces electrical biopotentials within the human body. These biopotentials decrease in amplitude with increasing distance from the active cell. The detection of biopotentials is therefore closely related to the detection of the ionic current created by the active cells. As modem electronics (i.e. EEG-amplifier) require electronic current, a biopotential electrode is a transducer transforming ionic current into electronic current. This current transformation is possible when an electrochemical electrode-electrolyte interface can be established (i.e. the human body fluids act as the electrolyte and a metallic biopotential electrode as the chemical electrode).

Skin anatomy is another important consideration in biopotential measurements. The skin presents a layered architecture. The outer skin layer, stratum corneum (SC), acts as a fluid barrier and therefore has electrical isolation characteristics. It also renders the administration of drugs on the skin less effective. This layer is constantly renewing itself and consists of dead cells. The stratum germinativum (SG), is the area where the cells divide, grow, and are displaced outward to the stratum corneum. Since the stratum germinativum is composed of living cells that predominately consist of liquid, this layer of the skin is an electrically conducting tissue comparable to an electrolyte. The dermis, which is below the stratum germinativum, contains vascular and nervous components as well as sweat glands and hair follicles and is also electrically conducting. It is in the dermis that pain has its origins.

When a metal electrode is applied on an unprepared surface of the skin, very high electrode-skin-electrode impedances (ESEI) result since no direct electrochemical electrode-electrolyte interface with the body fluids can be established. Therefore, conventional biopotential electrodes, require special skin preparation prior to application of the electrode. The two most common preparation methods are abrasion of the stratum corneum and the use of an electrolytic gel. The goal of abrasion is to reduce the thickness of the stratum comeum. However, complete removal of the stratum comeum is painful and not recommended. Alternatively, an electrolytic gel having a high concentration of conductive ions can be applied to the stratum corneum. The gel diffuses into the stratum corneum and improves its conductivity.

The combination of the above stated preparation methods present the possibility of reducing the ESEI by creating an electrode-electrolyte interface. However, important drawbacks of these methods are obvious: long application times, long stabilization times (diffusion of the electrolytic gel into the skin), long cleansing times (removal of the gel after use), and discomfort.

Biopotential electrodes that are not based on an electrochemical electrode-electrolyte interface have been described, for example, by Babak Alizadeh-Taheri et al., supra. These biopotential electrodes are active (i.e. signal amplification on the electrode) and use capacitive detecting principles. The main drawbacks of active electrodes are their high cost and complexity.

### BRIEF SUMMARY OF THE INVENTION

According to the invention, there is provided a method of forming a structure as defined by claim 1.

The present invention presents a new approach for a medical electrode to be applied on the skin of a living subject. The electrode can sense electric biopotentials, that is, the biopotentials being created within the body of the living subject and developed on the skin of the living subject, or can electrostimulate the skin and deeper lying tissue layers with electrical impulses.

The electrode formed by the present invention comprises a plurality of means, termed herein "spikes" to penetrate the skin. The spikes are long enough to reach the stratum germinativum and are able to carry an electrical signal. The spikes are electrically conductive on their surface and are connected to each other to form an array. The spikes are formed at the same time and from the same material as a plane carrier base member from which the spikes extend and, as the spikes are formed by etching a wafer of silicon or other material, the electrode may comprise a chip-like element.

Since the spike array of the biopotential electrode provides a means for penetrating the stratum corneum of a living subject, the electrode can serve as a means for making skin applied drug delivery more effective. For this purpose, a liquid retainer is provided on the carrier base member opposite to the surface on which the spikes are formed and supplied to the skin through a hole or holes in the base member.

Various other features, objects, and advantages of the invention will be made apparent from the following detailed description and the drawings.

### BRIEF DESCRIPTION OF THE DRAWING

In the drawing:
Fig. 1a is a cross-sectional view of a medical electrode with spikes according to the present invention;
Fig. 1b shows an alternative technique for fastening a lead wire to the electrode of the present invention;
Fig. 2 is a detailed view of a spike containing a barb to enhance retention of the electrode on the skin of the subject;
Fig. 3 is a detailed view of an array of spikes provided in the medical electrode of the present invention;
Figs. 4a-g show certain steps of a process for forming the medical electrode of the present invention;
Fig. 5 shows the deposition of a conductive layer by a process using a tilting and rotating mechanism;
Fig. 6 shows an embodiment of the medical electrode with a container for a drug to be delivered through the skin;
Fig. 6a shows, on an enlarged scale, a modified embodiment of the spikes suitable for use in the medical electrode shown in Fig. 6.
Fig. 7 shows a measurement circuitry for measuring the ESEI of biopotential electrodes;
Fig. 8 is a Nyquist plot showing the reduction in ESEI with the electrode of the present invention;
Fig. 9 is a further Nyquist plot showing the reduction in ESEI with the electrode of the present invention; and
Fig. 10 shows EEG output signals obtained with the electrode of the present invention and with a conventional electrode.

### DETAILED DESCRIPTION OF THE INVENTION

The medical electrode of the present invention comprises a base that includes an array of micro-dimensioned spikes designed to pierce the outer skin layer, i.e. the stratum corneum and penetrate into the electrically conductive stratum germinativum, thereby to circumvent the high impedance characteristics of the stratum corneum SC. However, the spikes must not reach the tissue layer below the stratum germinativum containing nerves and blood vessels so as to avoid pain or bleeding of the subject. The thickness of the stratum corneum is approximately 10 to 15 µm. The thickness of the stratum germinativum is about 50 to 100 µm. Thus, spikes that penetrate the skin more than 10-15 µm, but less than about 50-100 µm, produce a pain-free electrode-electrolyte interface at the stratum germinativum and transform the ionic current induced by active cells into an electronic current. To achieve this, experiments have shown that the spike length of a majority of the spikes in the array should be in the range of 150 to 350 µm, possibly as long as 500 µm.

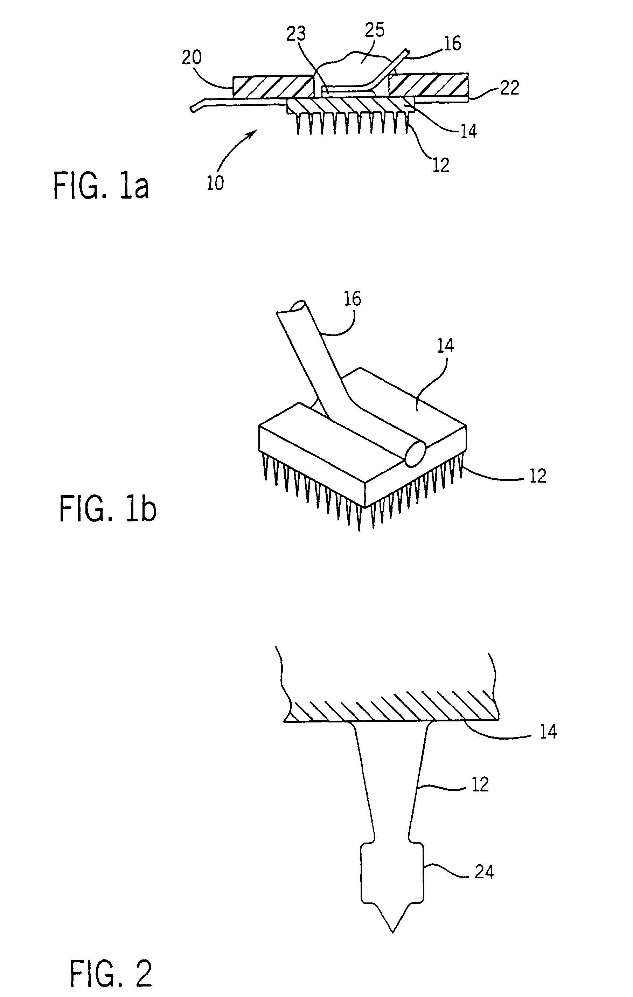

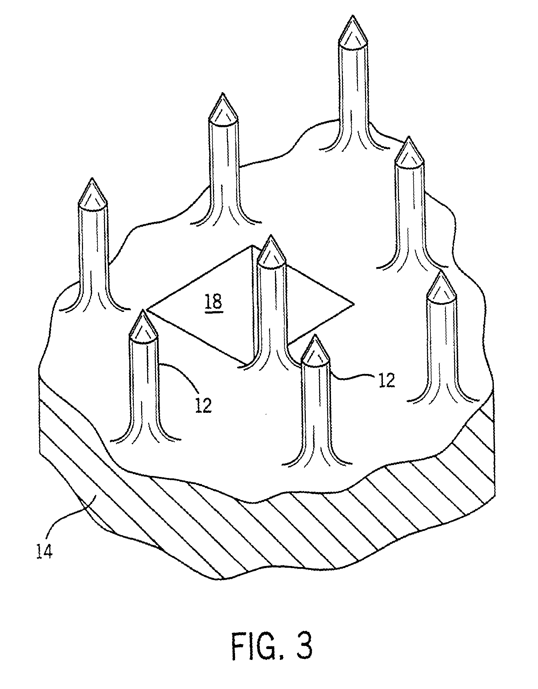

In medical electrode 10 of the present invention shown in Fig. 1, the bases of spikes 12 are joined to carrier base member 14 in a spaced array on the front side of the base member. Such an array is shown in Fig. 3. Fig. 3 shows an example in which spikes 12 are approximately 160 µm long and 40 µm wide. The spikes and the base member are, preferably, both made of the same material, for example as a micromachined silicon chip. All spikes 12 are interconnected and they are thus all at the same potential. In the alternative, a polymer base material may be used to reduce the cost of the electrode.

To reduce the most important noise source in an electrode-electrolyte interface, i.e. polarizability, the spikes should, preferably, be coated with a silver-silverchloride (Ag-AgCl) double layer in which only the AgCl is in contact with the electrolyte. See *Optimum Electrolytic Chloriding of Silver Electrodes*, L.A. Geddes, L.E. Baker and A. G. Moore, Med. & Biol. Engineering, Vol. 7, Pergamon Press, 1969. The advantages of silver and its compounds are low electrical resistivity and biomedical compatibility. Other conductive layers can also be used or if the spikes are made of a conducting material, they can be used without deposition of a conducting layer.

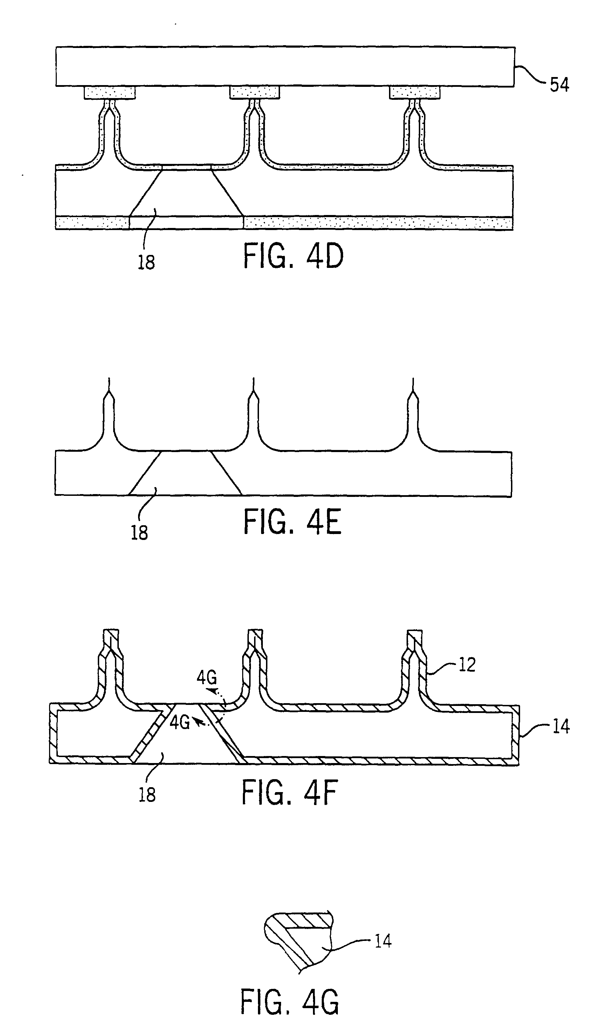

Medical electrode 10 is connected to analyzing electronic apparatus (not shown) by lead wire 16. As the wire diameter for lead wire 16 will be larger than the length of the spikes, it is preferably attached to the backside of electrode 10 to avoid any influence on the proper penetration of the spikes 12 into the skin. An electrical interconnection of the front side and backside of the electrode must be established. Therefore, a through-hole or holes 18, as shown in Fig. 3, can be fabricated and both sides of carrier base member 12 coated with silver. By paying special attention that the front side and backside conductive layer coating overlap, as shown in Fig. 4(f), the necessary connection may be made between spikes 12 and lead wire 16. Or, the connection may be obtained by overlapping conductive coatings along the edges of the base member.

In the dry use of the spiked medical electrode 10 of the present invention without an electrolyte gel or other skin preparation, the electrode should be securely attached to the skin of the subject to allow the spikes to pierce the stratum corneum and penetrate into the stratum germinativum. A simple structure for achieving this requirement comprises a thin, annular disk 20 fastened to the backside of carrier base member 14. The diameter of the disk 20 exceeds the dimension of base member 14. Ring-shaped adhesive tape 22 is fitted onto the disk and firmly fixes the disk and electrode 10 to the skin when applied to the skin. Lead wire 16 is connected to the backside of the electrode chip through a hole in the center of the disk as shown in Fig. 1a by conductive epoxy 23 and sealed with epoxy 25. Or, the lead wire can be glued by conductive glue or fixed by an appropriate clip fastener, as e.g. shown in Fig. 1b.

Attachment of medical electrode 10 to the skin of the subject can also be achieved by adding barbs 24 to the spikes as shown in Fig. 2, to directly attach the electrode to the skin. The use of barbed spikes can be combined with the adhesive tape attachment of Fig. 1a, if desired.

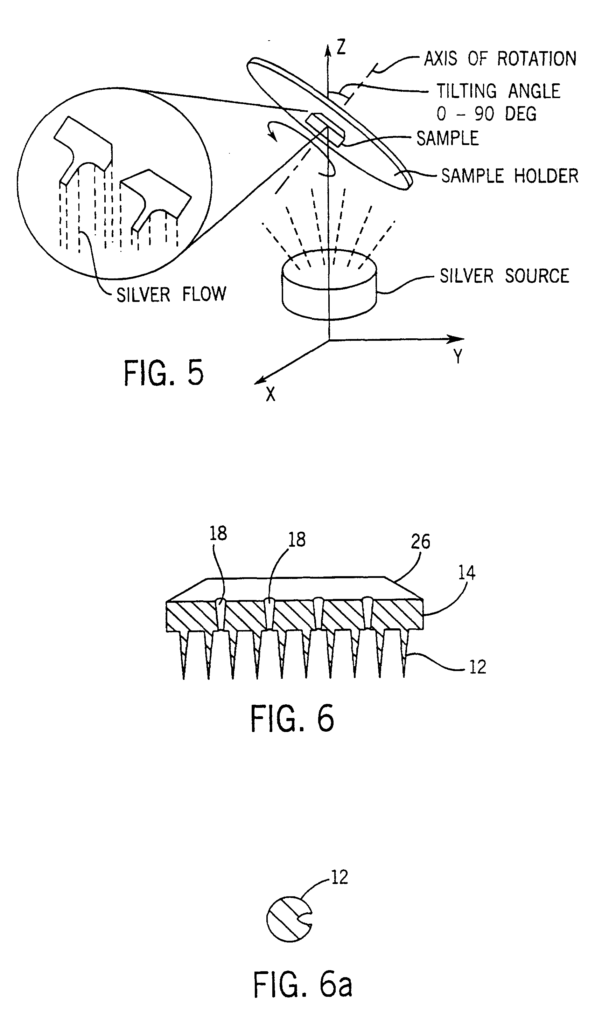

When attached to the skin of the subject, electrode 10 obtains biopotential signals or provides electrostimulation of body tissue. The electrode of the present invention may also be used to enhance the administration of skin applied drugs. To this end, a drug container, such as a flexible capsule 26, may be provided on the backside of the electrode, as shown in Fig. 6a. A plurality of through-holes 18 may be provided in base member 14 to discharge the drug to the skin of the subject when the flexible capsule is pressed. The penetration of the skin by the spikes facilitates passage of the drug through the stratum corneum. The length of the spikes in such an application also should be the same as for electrodes without such feature thus inflicting no pain to the patient. The drug delivery can be combined with electrical stimulation.

While the fabrication of these spikes for drug delivery can be realized with the round spikes shown in Fig. 3, they can also be realized by using channeled spikes, as shown in Fig. 6a. The enhanced delivery of the drug provided by the grooves is carried out by external pressure and/or capillary forces acting on the drug in the grooves. The external pressure may be created by pressing on flexible capsule 26.

Or, the spikes can be made hollow with the drug will be passed through through-holes in base member 14 into the spikes responsive to pressure on the capsule, similar to many small injection needles.

The fabrication of spikes extending from the plane of a silicon wafer has been reported in the technical literature. See for example *Micromachined Needles for the Transdermal Delivery of Drugs,* S. Henry, D.V. McAllister, M.G. Allen, and M.R. Prausnitz, Proceedings IEEE Micro Electro Mechanical Systems, 1998; *Novel AFM Probes-Fabrication and Characterization*, Anja Boisen, Ole Hansen and Siebe Bowastra, Micro Structure Workshop, 1998; and *Micromachined, Silicon Based Electrode Arrays for Electrical Stimulation of or Recording from Cerebral Cortex,* Richard A. Norman, Patrick K. Campbell and Kelly E. Jones, Proceedings IEEE Micro Electro Mechanical Systems, 1991.

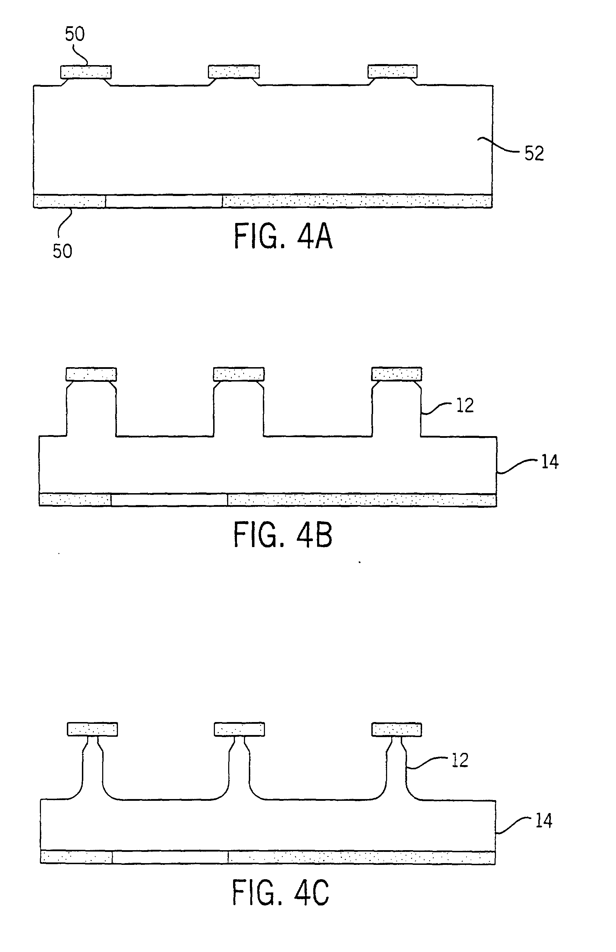

A feature of the present invention is that the spikes are formed from a wafer using a new three step deep reactive ion etching (DRIE) process. The steps of the process are shown in Figs. 4a-f. First, a circular oxide mask 50, the pattern of which is obtained using standard photolithography, is applied to a silicon wafer 52 and isotropically underetched (Fig. 4a). Second, an anisotropic process is carried out to define the length of spikes 12 and to form base member 14, as shown in Fig. 4b. Third, a second isotropic etch, which is stopped before the mask is completely underetched, smoothes the spikes and gives them a shape allowing easy penetration of the stratum corneum (Fig. 4c). As the underetching is not perfectly uniform, this step is stopped before the oxide masks of early finished spikes are completely underetched since if the oxide masks were to fall down and stick to the sidewalls of the spikes, the sharp tips of the spikes would be severely damaged. As shown in Fig. 4, this allows good individual control of the spike shape, length, and diameter.

For a given wafer, dimensions vary less than +/-2%. The length and diameter of the fabricated spikes typically range from 100 to 210 micrometers and 30 to 50 micrometers, respectively. Previously reported fabrication processes of spikes have not shown this individual control of making long tips (>150 micrometers) with high mechanical strength and tip sharpness.

To achieve barbed spikes, shown in Fig. 2, a combination of processes in a consecutive sequence of an isotopic and an anisotropic DRIE process, followed by an oxidation process, followed by an anisotropic and an isotropic DRIE process, followed by an oxide removal and a last isotropic DRIE process may be employed.

Through-holes 18 are KOH-etched in wafer 52 using a thermally grown oxide as a stop layer (Fig. 4d) and a (111) oriented wafer 54 glued with black wax as a front side protection. In addition to acting as a stop layer, the oxide can be used for a controlled removal of the circular oxide masks and for sharpening the spikes. By etching the oxide with HF; extremely sharp tip radii of less than 0.5 µm can be obtained (Fig. 4e).

A double-sided conductive layer deposition process employing a mechanical system that tilts and rotates the wafer (Fig. 5) may be used to coat the front- and backside of the wafer containing electrode 10 including spikes 12. This process allows overlapping of front- and backside coating at the through-hole while maintaining a uniform coating of the spike sidewalls. Hence, electrical interconnection of front side and backside as well as complete homogenous spike coating is obtained. The detailed showing of Fig. 4f demonstrates the overlapping of the two silver layers at a through-hole 18 and Fig. 3 shows the front side of a silver coated, spiked electrode.

Subsequently, AgCl is grown on the Ag via an uncomplicated electrochemical cell process, as described in Geddes, et al., supra. A 0.9 molar Cl⁻ solution in which a pure Ag reference electrode and the spiked electrode chip to be chlorided is dipped may be used for this purpose. By applying a constant current, the spiked electrode chip is chlorided. The control of current-density and process time controls the thickness and quality of the AgCI layer.

After dice sawing the wafer 52, the electrode chips are fastened onto e.g. plastic rings 20 with standard epoxy glue. Lead wire 16 is attached with conducting epoxy 23 and secured in electrode 10 with standard epoxy 25. The spiked electrode chips produced from wafer 52 can have a sizes of 1x1mm² to 5x5xmm².

The medical electrode and the spikes can also fabricated using a replication technique in this case the DRIE-etched spikes would be used for fabrication of the master for molding. The spikes are fabricated of a plastic material by an injection molding or hot embossing process.

A significant characteristic in achieving high performance from biopotential electrodes is low electrode-skin-electrode impedance (ESEI). Measurements were conducted to show that dry spiked electrodes, such as electrode 10 of the present invention, show ESEI as low as actually considerably lower than wet conventional electrodes. They were also found to be less complicated to use, faster to apply, and more comfortable for the subject.

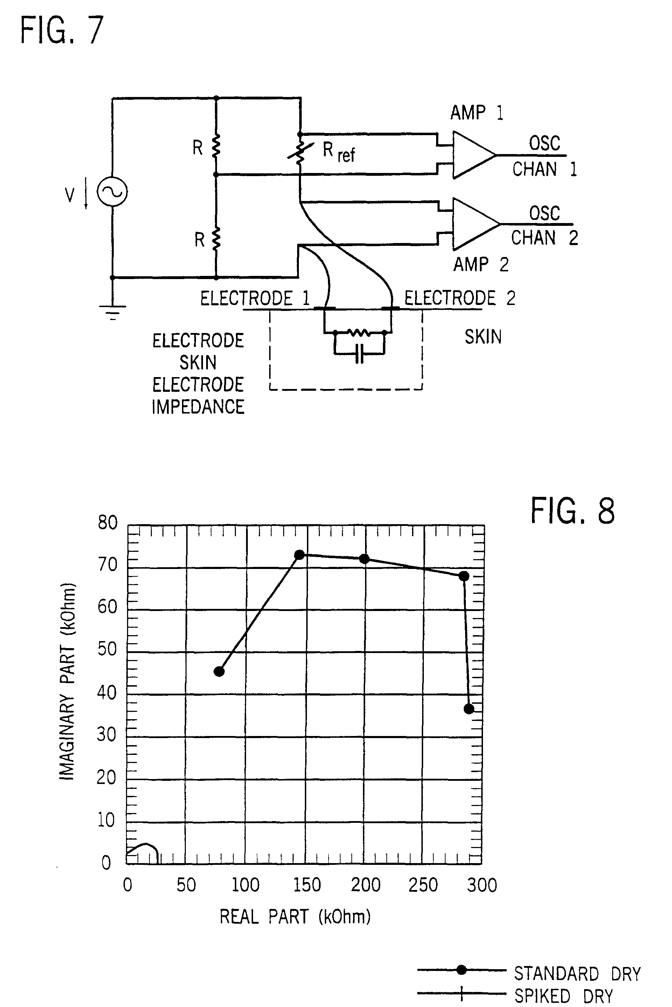

The measurement circuitry for evaluating the ESEI of biopotential electrodes is shown in Fig. 7. Signal v is a sinusoidal alternating voltage. R_{ref} is manually adjusted so the peak to peak value of the output voltage of Amp1 and Amp2 are equal. Knowing the temporal phase-shift of Amp1 and Amp2 and the value of R_{ref}, the ESEI (real and imaginary part) can be determined.

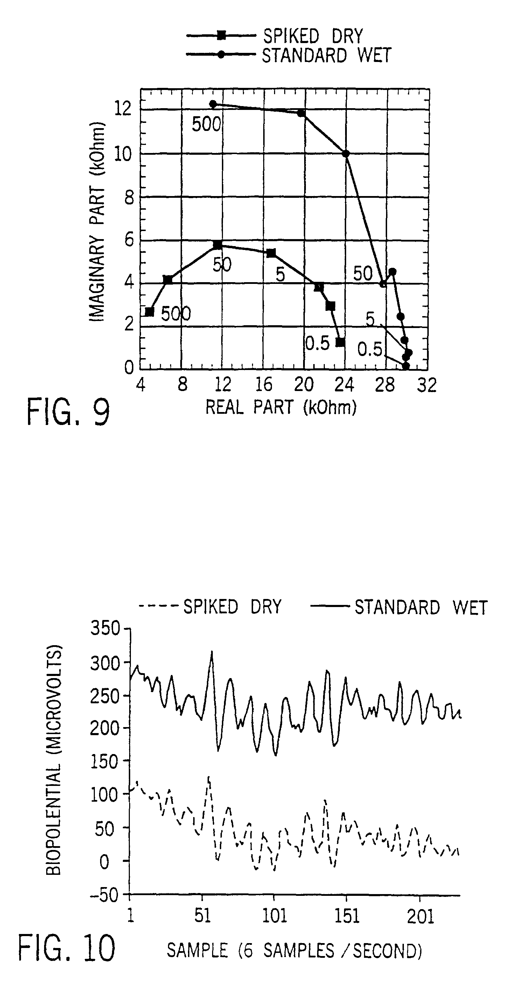

The ESEI is dependent on the measurement frequency. In the measurements discussed below and shown in Figs. 8 and 9, the frequency of v was chosen to range from 0.5 to 500 Hz which includes the interval of interest for biomedical applications. The RMS value of v is below 60mV so that it does not interfere with the membrane potential of active cells, so that there is no risk of harm for a test person, and so that current densities can be kept on a level as low as is comparable to currents during biopotential recordings. This is important since the electrical conductivity of the skin is dependent on the current density. Such low voltage levels imply that the measurements must be conducted in an environment devoid of extrinsic noise (e.g. in a Faraday Cage).

Standard EEG measurement recordings were conducted with spiked electrode of the present invention in sizes of 4x4mm² and 2x2mm². Electroencephalographic measurements exhibit the lowest biopotential levels and therefore need low ESEI to obtain reliable recordings of the brain activity.

Fig. 8 is Nyquist plot showing the influence of the spikes on the measured ESEI. The spiked electrodes of the present invention is compared with a standard electrode, applied without gel (i.e. dry) and without skin preparation. Fig. 8 clearly demonstrates the reduction of the ESEI by the action of the spikes by a factor of at least 13 when comparing a spiked electrode with a standard one applied without gel and skin preparation. The decreasing of impedance by the action of the spikes (both real and imaginary part) demonstrates clearly the advantages of spiked electrodes.

Fig. 9 is a Nyquist plot of the ESEI of a standard electrode using gel (standard wet) and of a spiked electrode of the present invention without gel (spiked dry). The numbers beside the curves indicate the measurement frequency. The spike electrode of the present invention clearly shows lower impedance than standard ones. In Fig. 9, the ESEI of dry spiked electrodes is reduced by a factor of at least 1.3 compared to standard electrodes using gel.

Fig. 10 represents recorded raw EEG data obtained with the spiked electrode of the present invention and with a wet standard electrode. Both recordings are very similar and show that the spiked electrode is suitable for EEG measurements. An EEG recording with eye movements was used to better show the similarity of both signals. The obtained electrical signals are stable with low noise levels even at low frequencies. The size of the spiked electrode chip can be reduced from 4x4mm² to 2x2mm² while maintaining very satisfactory results for these EEG measurements.

During use, the electrode of the present invention firmly attached to the skin and allowed the spikes to penetrate the skin. The spikes withstood even severe handling so that clinical as well as experimental use of the spiked electrode is possible. The application procedure for a micromachined electrode of the present invention is easy and fast while no pain was induced and stabilization times were shorter compared to standard electrodes.

Low ESEI, short stabilization times, and uncomplicated use without skin preparation are important characteristics of the spiked biopotential electrode and were confirmed during the experiments.

The experiment also confirmed that EEG recording is possible even with very small electrode sizes (2x2mm²). The fact that low signals of EEG can be sensed indicates, that the spiked electrode can be used for other biomedical applications since, in general, they have stronger signals. No standard biopotential electrodes reliably measuring biopotentials on the surface of the skin and showing dimensions as small as 2x2mm² have been found by the inventors. ESEI measurements for 2x2mm² electrodes are not meaningful since current densities are higher than for actual biopotential recording.

It is recognized that other equivalents, alternatives, and modifications aside from those expressly stated, are possible and within the scope of the appended claims.

## Claims

1. A method of forming a structure having a plurality of spikes extending from a carrier base member, the lengths of the spikes ranging from 150 to 500µm, said method comprising the steps of:
providing a wafer of material (52);
applying an etchant resistant mask (50) to a surface of the wafer, the mask defining the spikes of the structure and having a pattern establishing the location of the spikes on the carrier base member;
carrying out a first process that is essentially only an isotropic etching of the surface of the wafer to undercut the mask along the surface of the wafer;
carrying out a second process that is essentially only an anisotropic etching of the surface of the wafer to remove an amount of material from the unmasked portion of the wafer corresponding to the length of the spikes (12) and to form the remainder of the wafer into the carrier base member (14) of the structure; and
carrying out a third process that is essentially only an isotropic etching of the exposed portion of the wafer to define spikes having tapering ends.

2. The method of claim 1 further comprising the steps of applying an etchant resistant stop layer to the spikes and carrier base member formed from the wafer and etching the stop layer at the tapered ends of the spikes.

3. The method of claim 2 further defined as applying a thermally grown oxide etchant resistant stop layer to the spikes and carrier base member.

4. The method of claim 2 further defined as etching the stop layer at the tapered ends of the spikes using hydrofluoric (HF) acid.

5. The method of claim 1 further comprising the steps of applying an etchant resistant stop layer to the carrier base member formed from the wafer, the stop layer defining a pattern for through-holes, and etching through-holes in the portion of

6. The method of claim 5 further defined as etching the through-holes using KOH.

7. The method of claim 1 further comprising the step of coating at least the spikes and the side of the carrier base member from which the spikes extend with a conductive layer.

8. The method of claim 7 wherein the step of coating with a conductive layer is further defined a coating with a silver layer.

9. The method of claim 7 further defined as creating a silver chloride layer on the silver layer.

10. The method of claim 1 further defined as forming the structure through the step of dicing the wafer.

11. The method of claim 5 further defined as providing a fluid container and a hole in the carrier base member for transferring fluid through the through-hole in the carrier base member.

12. The method of claim 1 further defined as providing a fluid container on the carrier base member and forming fluid passageways in the spikes for transferring fluid through or along the spikes.

13. The method of claim 12 further defined as forming grooves in the spikes for transferring fluid along the spikes.

14. The method of claim 13 further defined as forming holes in the spikes for transferring fluid through the spikes.

15. The method of claim 1 through 13 further defined as a method for forming a structure comprising one of a medical electrode device, a medical liquid transfer device, or a replication master for such devices.

## Patentansprüche

1. Ein Verfahren zum Bilden einer Struktur, die eine Mehrzahl von Spitzen aufweist, die sich von einem Trägerbasisbauglied erstrecken, wobei die Längen der Spitzen zwischen 150 und 500 µm liegen, wobei das Verfahren die folgenden Schritte aufweist:
Bereitstellen eines Wafers aus einem Material (52);
Aufbringen einer ätzmittelresistenten Maske (50) auf eine Oberfläche des Wafers, wobei die Maske die Spitzen der Struktur definiert und ein Muster aufweist, das die Position der Spitzen an dem Trägerbasisbauglied einrichtet;
Ausführen eines ersten Prozesses, der im Wesentlichen lediglich ein isotropisches Ätzen der Oberfläche des Wafers ist, um die Maske entlang der Oberfläche des Wafers zu unterschneiden;
Ausführen eines zweiten Prozesses, der im Wesentlichen lediglich ein anisotropisches Ätzen der Oberfläche des Wafers ist, um eine Menge an Material von dem unmaskierten Abschnitt des Wafers entsprechend der Länge der Spitzen (12) zu entfernen und den Rest des Wafers in das Trägerbasisbauglied (14) der Struktur zu bilden; und
Ausführen eines dritten Prozesses, der im Wesentlichen lediglich ein isotropisches Ätzen des freiliegenden Abschnitts des Wafers ist, um Spitzen zu definieren, die sich verjüngende Enden aufweisen.

2. Das Verfahren gemäß Anspruch 1, das ferner die Schritte eines Aufbringens einer ätzmittelresistenten Stoppschicht auf die Spitzen und das Trägerbasisbauglied, das aus dem Wafer gebildet ist, und eines Ätzens der Stoppschicht bei den sich verjüngenden Enden der Spitzen aufweist.

3. Das Verfahren gemäß Anspruch 2, das ferner definiert ist als ein Aufbringen einer thermisch aufgewachsenen oxidätzmittelresistenten Stoppschicht auf die Spitzen und das Trägerbasisbauglied.

4. Das Verfahren gemäß Anspruch 2, das ferner definiert als ein Ätzen der Stoppschicht bei den sich verjüngenden Enden der Spitzen unter Verwendung von Fluorwasserstoffsäure (HF-Säure).

5. Das Verfahren gemäß Anspruch 1, das ferner die Schritte eines Aufbringens einer ätzmittelresistenten Stoppschicht auf das Trägerbasisbauglied, das aus dem Wafer gebildet ist, wobei die Stoppschicht ein Muster für Durchgangslöcher definiert, und ein Ätzen von Durchgangslöchern in dem Abschnitt des Wafers, der das Trägerbasisbauglied bildet, aufweist.

6. Das Verfahren gemäß Anspruch 5, das ferner definiert ist als ein Ätzen der Durchgangslöcher unter Verwendung von KOH.

7. Das Verfahren gemäß Anspruch 1, das ferner den Schritt eines Beschichtens zumindest der Spitzen und der Seite des Trägerbasisbauglieds, von dem sich die Spitzen erstrecken, mit einer leitfähigen Schicht aufweist.

8. Das Verfahren gemäß Anspruch 7, bei dem der Schritt des Beschichtens mit einer leitfähigen Schicht ferner als ein Beschichten mit einer Silberschicht definiert ist.

9. Das Verfahren gemäß Anspruch 7, das ferner als ein Erzeugen einer Silberchloridschicht auf der Silberschicht definiert ist.

10. Das Verfahren gemäß Anspruch 1, das ferner als ein Bilden der Struktur durch den Schritt eines Vereinzelns des Wafers definiert ist.

11. Das Verfahren gemäß Anspruch 5, das ferner definiert ist als ein Bereitstellen eines Fluidbehälters und eines Lochs in dem Trägerbasisbauglied zum Übertragen eines Fluids durch das Durchgangsloch in dem Trägerbasisbauglied.

12. Das Verfahren gemäß Anspruch 1, das ferner definiert ist als ein Bereitstellen eines Fluidbehälters an dem Trägerbasisbauglied und ein Bilden von Fluiddurchgängen in den Spitzen zum Übertragen von Fluid durch die oder entlang den Spitzen.

13. Das Verfahren gemäß Anspruch 12, das ferner definiert ist als ein Bilden von Rillen in den Spitzen zum Übertragen von Fluid entlang den Spitzen.

14. Das Verfahren gemäß Anspruch 13, das ferner definiert ist als ein Bilden von Löchern in den Spitzen zum Übertragen von Fluid durch die Spitzen.

15. Das Verfahren gemäß Anspruch 1 bis 13, das ferner als ein Verfahren zum Bilden einer Struktur definiert ist, die eine medizinische Elektrodenvorrichtung, eine medizinische Flüssigkeitsübertragungsvorrichtung oder einen Replikationsmaster für derartige Vorrichtungen aufweist.

## Revendications

1. Procédé pour former une structure munie d'une pluralité de pointes s'étendant depuis un organe de base porteur, les longueurs des pointes mesurant de 150 à 500 µm, ledit procédé comprenant les étapes consistant à :
prévoir une plaquette de matériau (52) ;
appliquer un masque (50) résistant à l'agent de gravure sur une surface de la plaquette, le masque définissant les pointes de la structure et ayant un motif établissant l'emplacement des pointes sur l'organe de base porteur ;
exécuter un premier processus qui est essentiellement seulement une gravure isotrope de la surface de la plaquette pour évider le masque le long de la surface de la plaquette ;
exécuter un deuxième processus qui est essentiellement seulement une gravure anisotrope de la surface de la plaquette pour enlever une quantité de matériau de la portion sans masque de la plaquette correspondant à la longueur des pointes (12) et pour former le reste de la plaquette dans l'organe de base porteur (14) de la structure ; et
exécuter un troisième processus qui est essentiellement seulement une gravure isotrope de la portion mise à nue de la plaquette pour définir des pointes munies d'extrémités allant en s'effilant.

2. Procédé selon la revendication 1 qui comprend en outre les étapes consistant à appliquer une couche d'arrêt résistante à l'agent de gravure sur les pointes et l'organe de base porteur formés depuis la plaquette et à graver la couche d'arrêt au niveau des extrémités effilées des pointes.

3. Procédé selon la revendication 2 défini en outre comme appliquant une couche d'arrêt résistante à l'agent de gravure oxydée par tirage thermique sur les pointes et l'organe de base porteur.

4. Procédé selon la revendication 2 défini en outre comme gravant la couche d'arrêt au niveau des extrémités effilées des pointes en utilisant de l'acide hydrofluorhydrique (HF).

5. Procédé selon la revendication 1 qui comprend en outre les étapes consistant à appliquer une couche d'arrêt résistante à l'agent de gravure sur l'organe de base porteur formé depuis la plaquette, couche d'arrêt définissant un motif pour des trous traversants, et à graver les trous traversants dans la portion de la plaquette formant l'organe de base porteur.

6. Procédé selon la revendication 5 défini en outre comme gravant les trous traversants en utilisant du KOH.

7. Procédé selon la revendication 1 qui comprend en outre l'étape consistant à revêtir d'une couche conductrice au moins les pointes et la face de l'organe de base porteur d'où les pointes s'étendent.

8. Procédé selon la revendication 7 dans lequel l'étape consistant à revêtir d'une couche conductrice est définie en outre comme étant un revêtement avec une couche d'argent.

9. Procédé selon la revendication 7 défini en outre comme créant une couche de chlorure d'argent sur la couche d'argent.

10. Procédé selon la revendication 1 défini en outre comme formant la structure en passant par l'étape de découpage en cubes de la plaquette.

11. Procédé selon la revendication 5 défini en outre comme prévoyant un contenant de fluide et un trou dans l'organe de base porteur pour transférer le fluide à travers le trou traversant dans l'organe de base porteur.

12. Procédé selon la revendication 1 défini en outre comme prévoyant un contenant de fluide sur l'organe de base porteur et formant des passages pour fluide dans les pointes pour transférer le fluide à travers les pointes ou le long de celles-ci.

13. Procédé selon la revendication 12 défini en outre comme formant des sillons dans les pointes pour transférer le fluide le long des pointes.

14. Procédé selon la revendication 13 défini en outre comme formant des trous dans les pointes pour transférer le fluide à travers les pointes.

15. Procédé selon les revendications 1 à 13 défini en outre comme étant un procédé pour former une structure comprenant soit un dispositif à électrode médicale, un dispositif de transfert de liquide médical, ou un modèle de reproduction pour de tels dispositifs.
